# EUROPEAN PATENT SPECIFICATION

(11) **EP 2 928 419 B1**
(45) Date of publication and mention of the grant of the patent: **25.10.2017**
(21) Application number: 13860613.2
(22) Date of filing: 06.12.2013
(51) Int. Cl.: A61F 2/64, A61F 2/68, A61F 2/70, A61F 2/76

(54) **KNEE JOINT PROSTHESIS**
KNIEGELENKPROTHESE
PROTHÈSE D'ARTICULATION DE GENOU

(30) Priority: 06.12.2012 SE 1251379
(43) Date of publication of application: 14.10.2015
(73) Proprietor: CENTRI AB, 191 27 Sollentuna (SE)
(72) Inventor: HELLBERG, Kennet, 186 33 Vallentuna (SE); RANDSTRÖM, Alexander, 112 43 Stockholm (SE)
(74) Representative: Bergenstråhle Group AB
(86) International application number: PCT/SE2013/051464
(87) International publication number: WO 2014/088505

(56) References cited:
- EP-A1- 1 169 982
- US-A- 1 834 491
- US-A- 5 895 430
- US-A1- 2002 193 887
- US-A1- 2004 181 289
- US-A1- 2009 265 018
- US-B2- 7 736 394
- US-B2- 8 231 688

## Description

### Technical field

The present invention relates to the field of knee joint prostheses.

### Background art

When a person is amputated above the knee a lower limb prosthesis comprising a prosthesis knee joint is usually needed. When the lower limb prosthesis is manufactured a casting of the amputation stump is made, serving as a template for the custom socket, i.e. the part that is attached to the amputation stump.

Today the amputation stump is kept as long as possible when a limb is amputated. The reason for this is that it is easier to control the prosthetic limb with a longer residual limb. A longer residual limb can withstand a higher load from the prosthetic limb than a shorter one. It is important that the amputation stump is as long as possible because then the load will be distributed on a larger body surface area, and that will reduce the pressure and shear forces exerted on the amputation stump.

More and more amputees are amputated through the knee through knee disarticulation amputation. This gives a very long amputation stump compared to other forms of amputation, which is better for control of the movements because of having a bigger surface area. All currently available prosthetic joints perform flexion and extension movements. A disadvantage with current methods for providing a prosthesis is that knee joints used today are relatively large, which can be inconvenient for the amputee in many ways, for example by distorting posture or hindering movements.

Today's knee joint prostheses are relatively heavy compared to natural knee joints. The artificial knee joint is surrounded by a support with one attachment arm placed on the lateral side and one on the medial side. One problem with this design is that the socket is always a little bit wider than the knee joint prosthesis and will be placed proximal to the knee joint prosthesis. This means that the knee joint prosthesis ends up in the wrong place if the residual limb/amputation stump is long. A knee joint prosthesis that is in the wrong place looks strange and it is difficult to make it work in an anatomically correct manner.

These prosthetic joints use dampening devices such as pneumatic and hydraulic cylinders, magnetic particle fluid brakes and friction to dampen energy generated during the flexion and extension of the prosthetic joint. Such a device works like a pendulum that is dampened during normal use. Knee joints using cylinders controlled by a valve brake the motion of the leg when the leg is straightened. When the leg is to be bent, energy is needed to perform the bending movement. This means that the lower limb and the foot must be of at least some weight since it is the oscillating energy, i.e. the motion of inertia that brakes the motion, thereby controlling the movement of the knee joint. If the lower limb and/or foot is too light, the pendulum will not gain enough energy to sufficiently brake the motion.

Even if the material and design for prostheses are improved today compared to before, through ways such as low weight materials and a more natural design, the mechanism of controlling a joint is still built on a pendulum movement. One of the problems today is that if the lower limb prosthesis and/or prosthesis foot have too low weight they cannot properly function as a pendulum providing sufficient kinematic energy to dampen the movement. In addition, friction from clothes and from the cosmetics surrounding the prosthesis further impedes movement of the pendulum, causing the person to need even more inertia.

There are also times when an amputee needs to extend or flex the knee joint prosthesis. For example, if the amputee wants to pass an obstacle he or she has to first bend the leg and thereafter put the leg forward over the obstacle, then extend the knee joint prosthesis and thereafter put it down on the other side of the obstacle. Doing this in a slow and controlled manner can be difficult for an amputee using a prosthesis, and is impossible to do with an above the knee, AK, prosthesis that only dampens the knee joint. It can also be difficult to walk at a slow pace, in a hill or in stairs.

A seminal patent application describing a hydraulic knee joint design for artificial legs was filed in 1948 (US 2,519,226). After that, only minor modifications have been made.

Many attempts to improve the control of knee joint prosthesis have been made, both with basic and electronically controlled joints.

Some knee joints are controlled a by hydraulic dampener. For example, US 6,106,560 describes a patient-weight-activated hydraulic knee joint, in which flow between hydraulic chambers is controlled by a valve operated by hydraulic pressure and by a force transmission system which transmits the force of a patient's weight. US2010/0292807 describes a stabilizing weight activated knee joint. AU2003200929 describes a knee joint prosthesis with a hydraulic dampening cylinder for regulating the phase control and stance phase stabilization and with an electronic control for force field acting on the hydraulic fluid of the dampening cylinder.

Many of the prosthetic knee joints controlled by microprocessors are passive where the prosthetic joint has an actuator valve controlling a hydraulic cylinder or a pneumatic cylinder dampener with a microprocessor controlled actuator valve changing the dampening. It is well documented how this type of prosthetic dampener should work to get the best swing and stance phase control for normal walking in even terrain. How the bending and/or stretching resistance is changed depends on sensor data. US2012215323 describes a device and method for controlling an artificial orthotic or prosthetic joint of a lower extremity.

However, the microprocessor controlled knee joints known today are relatively heavy compared to natural joints and do not take into account whether the amputation stump is long or short. Depending on the length of the amputation stump the knee joints may have significant height differences, which may bring unnecessary attention to a user.

Active knee joint prostheses have been developed in an attempt to address problems associated with known passive knee joint prostheses. One problem with active knee joint prostheses is that they all aim for a robotic walking style. They are heavy and strong and consume a lot of energy. Some implementations have the possibility to restore energy from the dampening activity during walk, but is still a knee joint prosthesis with a heavy frame, a heavy joint and a heavy dampening device.

US7485152 describes a prosthetic leg having an electronically controlled regenerative prosthetic knee. The prosthetic knee makes use of an actuator motor/generator to control gait and/or to generate electrical energy. An electronic control system is provided to control overall operation of the prosthetic leg, to distribute generated electrical energy, and to transfer excess electrical energy to one or more storage devices for later use. However, this electronically controlled regenerative prosthetic knee has the same problem as the ones described above due to a heavy frame and the use of a dampener system.

US 2002/0193887 discloses a lock adapter adapted to connect a residual limb liner with locking pin to a transfemoral prosthetic limb. The lock adapter is formed of a bracket having a first end and a second end, and preferably has a generally S-shaped configuration. The first end of the bracket has a first mounting face against which the lock is to be secured. The second end of the bracket is provided with a second mounting face against which the prosthetic limb is to be secured. The bracket being is formed so that the first and second mounting faces are spaced apart from one another, and may optionally be angled relative to one another.

US 5,895,430 discloses a prosthesis for leg amputation at the knee joint or short distance above the knee which includes an upper leg socket and artificial lower limb pivotally coupled together by a knee bracket attached to upper leg socket. The knee bracket is also pivotally coupled to a hydraulic unit carried by the lower limb. The knee bracket provides a hinge arrangement for the knee joint above the end of the amputee's leg stump by several inches.

The knee joint prostheses of prior art also suffer from other drawbacks. For example, it has been found that due to the significant energy input required, very few AK-amputees are able to ascend stairs in a leg over leg manner using a prosthetic leg having a passive knee joint. Simply put, due to the lack of a natural knee joint and corresponding muscles, most amputees lack the strength necessary to ascend stairs in a conventional manner using a prosthetic leg having a knee joint prosthesis available today. The medial and lateral bearings determine the width of the knee joint prosthesis, and since all amputation stumps are different it can be difficult to make a nice transition between the custom-made socket and the knee joint prosthesis. Ii is also important that clothes do not get stuck when the prosthetic knee moves, because then the clothes can break which can cause the function of the knee joint prosthesis to be poor.

Yet another problem is that when an amputee having a knee joint prosthesis sits down, the thigh portion of the knee joint prosthesis will add to the residual thigh. If the residual thigh is too long then the total length of the thigh will also become too long and the knee joint prosthesis will be located too proximal to the shin.

Another problem with currently available prosthetic knee joints of this kind is the weight. Most people in need of prostheses are over 65 years old. When the prostheses are very heavy they make it difficult for the person to obtain a natural-looking gait. The weight is also a problem for people who are muscle weak, disabled, sick, elderly and/or children.

### Summary of the invention

An object of the present invention is to provide a solution that counteracts some of the problems found in in prior art, thereby facilitating the walk for a person having a lower limb prosthesis. It is important that a prosthesis is of as low weight as possible and that the weight is located as proximally as possible. This minimizes the energy that the amputee needs to use to move the prosthetic leg, which is one problem that the present invention overcomes.

According to a general aspect of the invention a knee joint for use in a lower limb prosthesis comprises
- an upper portion comprising an attachment part with a first connection means for connecting a socket, and a support comprising a second connection in a lower part thereof for connecting a prosthetic limb,
- a joint, pivotably interconnecting the upper portion and the support, and
- an actuator connected between a proximal attachment and the support, and adapted to pivot the upper portion relatively to the support, characterized in that the joint is provided above the first connection means when the lower limb prosthesis is in normal use.

In some embodiments the knee joint of the present invention may be directly controlled by a motor, such as an electric motor. The electric motor can be for instance a linear motor, a belt motor or a hollow shaft motor.

Some embodiments the knee joint has a single sided support comprising a single attachment arm on either the medial or the lateral side of the knee joint prosthesis. A knee joint with a single side attachment arm is very easy to fit cosmetically.

According to a second aspect of the invention there is provided a method for controlling a knee joint as described above comprising the following steps; receiving data from the control unit; and - controlling the at least one actuator in dependence of the received data; and controlling the motor to consume or generate energy depending on the received data and/or controlling the angular velocity of the knee joint in dependence of the received data.

Another important advantage of the present invention is that the prosthetic knee fits amputees with long residual limbs, such as AK amputees - a method that is preferred today. Another important advantage is that the present invention is also compatible with a lower limb prosthesis using a low weight prosthesis foot. A problem for knee joint prostheses found in prior art is that a sufficient pendulum motion is not achieved when the pendulum, for instance a foot, does not have a sufficient weight. The absence of the need of a pendulum motion makes the present knee joint prosthesis superior to prior art.

The present invention is compatible with the use of low weight prosthesis and at the same time has a natural-looking appearance and a function that mimics the natural movements of an artificial leg. These are some of the problems of the prior art solved by the present invention.

The advantages with a knee joint prosthesis according to the present invention are many. Firstly the support surrounding the knee joint prosthesis is much lighter compared to those found on the market today, making movements easier for the amputee. Secondly, the prosthesis may be fitted more exact in relation to the corresponding place for the natural knee, something that is not possible today. The knee joint prosthesis center is situated below the level of the other natural knee-cap, i.e., the leg that is not amputated. The difference in height influences the gait and might seem odd for other people.

By combining the aspect of a knee joint prosthesis that uses a single sided support with the placement of the joint above the first connection means, the present invention offers a prosthesis that is smaller, lighter and easier to control than previous implementations. The aspect of an external controller with different modes that can be manually controlled by the user provides the user with more freedom and adaptability than previously possible.

The present invention provides a solution that counteracts some of the problems in prior art, thereby facilitating the walk for a person having a leg prosthesis. By controlling the prosthesis knee joint by a motor makes the movement more smooth and therefore the person's gait more natural. Moreover, the present invention also provides a prosthesis knee joint that is much lighter in weight compared to those found in prior art. The present invention also facilitates the walk for persons wearing a prosthetic lower leg, which is especially important for people who are muscle weak, disabled, sick, elderly and/or children.

Further preferred embodiments are defined in the dependent claims.

### Brief description of drawings

The invention is now described, by way of example, with reference to the accompanying drawings, in which:
Figs. 1 a-b show a side view and a perspective view of a knee joint.
Fig. 2a shows a view of an actuator, fig.2b shows a more detailed view of the same actuator and fig. 2c shows a perspective view of a proximal part of a lower limb prosthesis comprising a knee joint.
Figs. 3 a shows a view of an actuator and detailed views of different active direct electric actuators that control the angular velocity of the knee joint are shown in Figs. 3b-d.
Fig. 4 shows a diagram describing the operation of a knee joint of the present invention using an electric control system.
Fig 5 shows an external control device that can be used to manually select different modes for a knee joint.

### Description of embodiments

Before the invention is disclosed and described in detail, it is to be understood that this invention is not limited to particular materials or configurations disclosed herein as such configurations and materials may vary. It is also to be understood that the terminology employed herein is used for the purpose of describing particular embodiments only and is not intended to be limiting.

In the context of the present invention the words person, amputee and user are used interchangeably for the person who is going to use the prosthesis, i.e., the amputated person.

In the context of the present invention the words amputation stump or stump are used interchangeably for the portion of the amputated extremity that is left to serve as an attachment for a suitable prosthesis.

In the context of the present invention the custom socket is substantially the part of the prosthesis that is attached to the amputation stump. The custom socket is shaped to fit the conical shape of the amputation stump.

In the context of the present invention AK-amputee stands for amputation above the knee and BK-amputee stands for amputation below the knee

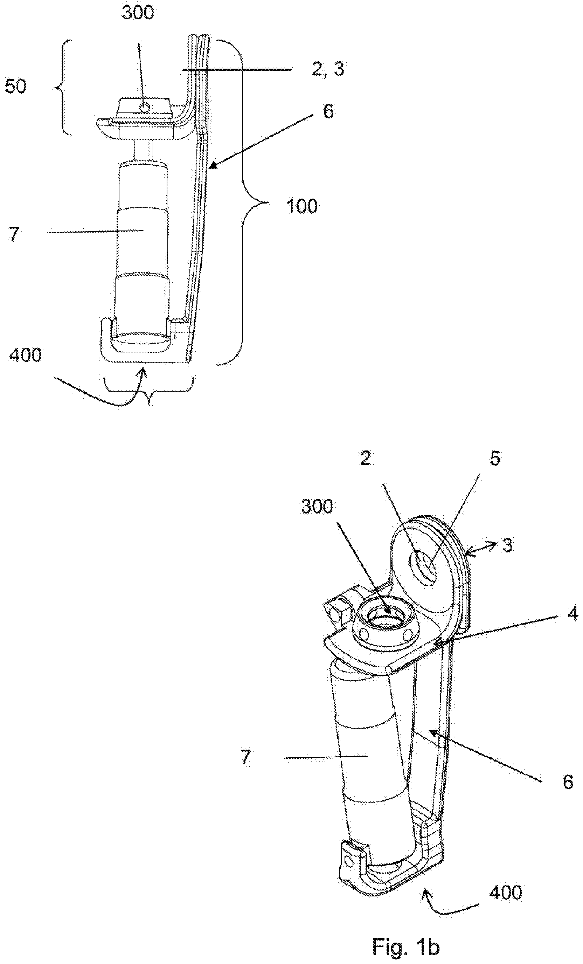

Fig. 1 a shows a side view of a knee joint of the present invention. The knee joint comprises an upper portion 50 connectible to a socket via a first connection means 300, and a support 100 connectible to a prosthetic limb via a second connection means 400 situated in its lower portion 75. The upper portion 50 and the support 100 are pivotably connected to each other via a joint 2, 3. An actuator 7 is connected between the upper portion 50 and support 100 of the knee joint. The support 100 may be single or double sided, i.e. have one or two attachment arm(s) 6. The embodiment shown in the figure only has one attachment arm 6 and is therefore single sided. The actuator in this embodiment has a cylindrical shape. However, the shape is not limited to cylindrical shapes but may be of optional shape as long as the actuator may perform its work.

Fig. 1b shows a perspective view of the same embodiment of the knee joint shown in fig. 1 a. The design of the knee joint is more pronounced in this figure. The upper portion 50 having a first connection means 300 comprises a proximal attachment 4, and a knee joint center 5. A joint 2, 3 connects the upper part 50 to a support 100 comprising a longitudinal attachment arm 6 and a clamp-like bottom portion 75. The actuator 7 is connected between the clamp-like portion 75 and the proximal attachment 4.

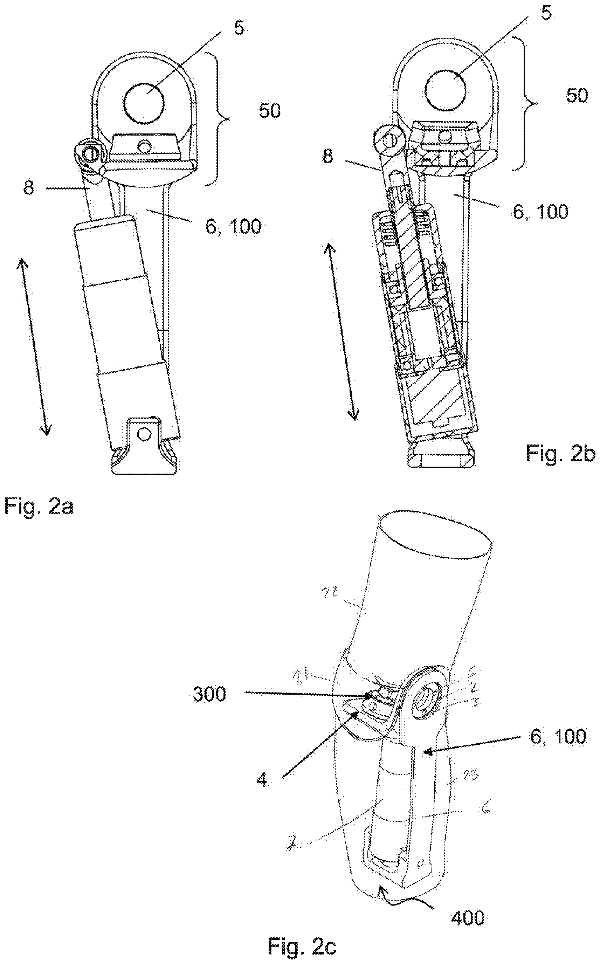

Fig 2a shows a view of the actuator 7 wherein a shaft 8 connecting to the actuator is in a somewhat extended position. Fig. 2b shows the same actuator but in a more detailed view without housing. The knee is in this case in a straight position.

Fig. 2c shows a view of the proximal part of a prosthesis according to the present invention, depicting one specially adapted socket 22 and a split cosmetics thin shell adapted to reduce friction in the knee joint when bending a lower limb prosthesis. The function of the split cosmetic shell is that it produces less friction when the leg is bent compared to a whole cosmetic shell, because the split cosmetics do not have to be stretched out. However, the prosthesis and cosmetics can be made of various materials and colors suitable for their purpose. The prosthesis comprises in its upper portion 50 a custom socket 22 connected to the first connection means 300 of the knee joint. The socket 22 is adapted to be fitted to an amputee's leg. The second connection means 400 is situated in the lower part 75 of the support 100 for being attached to an additional part of a lower limb such as a tibia or a foot prosthesis. A proximal portion 21 of the cosmetic shell covers the connection between the custom socket 22 and the lower part of the prosthesis covered by a lower cosmetic shell 23, where only the distal portion 23 is shown in the figure. The cosmetic shell covers the prosthesis so it looks more real and natural so as not to attract attention.

The upper portion 50 of the knee joint is connected to the support 100 via joint comprising an inner ring 2 and an outer ring 3. In a preferred embodiment the joint is provided above the first connection means 300 when the lower limb prosthesis is in normal use. This kind of embodiment enables prostheses to be compatible with longer amputation stumps than previously possible, while still being compatible with short ones as well. The type of joint is not limiting to the invention, and could for example be; a slide bearing, a cross roller bearing, a cross needle bearing, and ball bearing with a deep groove, which takes up high axial and radial forces.

The support 100 is in a preferred embodiment in the form of at least one vertical attachment arm 6 and a lower horizontal part 75 with a clamp-like shape. The support 100 can also have multiple attachment arms. The support 100 is fastened to an inner bearing ring 2 forming the prosthetic knee proximal attachment 4 located distal to the knee joint center 5, and the distal attachment arm 6 is located distal to the actuator 7. The support 100 comprising the attachment arm 6 and the lower part 75 may be attached in many different ways known to those skilled in the art. The knee joint in the shown embodiment only has one attachment arm 6.

One of the many advantages of having only one attachment arm 6 is that such a knee joint has a reduced weight and size compared to other knee joints having two or more attachment arms. The attachment arm 6 may be situated on either the medial or the lateral side of the knee joint prosthesis. Moreover, the features of the present invention enhance and facilitate the walk for the amputee due to the reduced weight as well as the reduced size of the prosthesis knee joint. It also makes it easier to put the leg in a more correct position and to control the flow of the gait. Moreover, the small size of the present knee joint prosthesis compared to previously known alternatives makes it easier to fit in the right place of the knee portion which also makes the lower limb prosthesis more convenient. It is also important that a knee joint prosthesis has as low weight as possible, and that the weight is located as proximally as possible. This minimizes the energy that the amputee needs to move the prosthetic leg and is also important for the function of the present knee joint prosthesis. For example, a knee joint prosthesis with a relatively heavy weight would significantly impact the center of mass of the artificial leg of an amputee using it, moving the center downward towards the foot section. Like any pendulum, the prosthetic leg would then require more energy to move than one with a center of mass located closer to the natural placement of a knee joint.

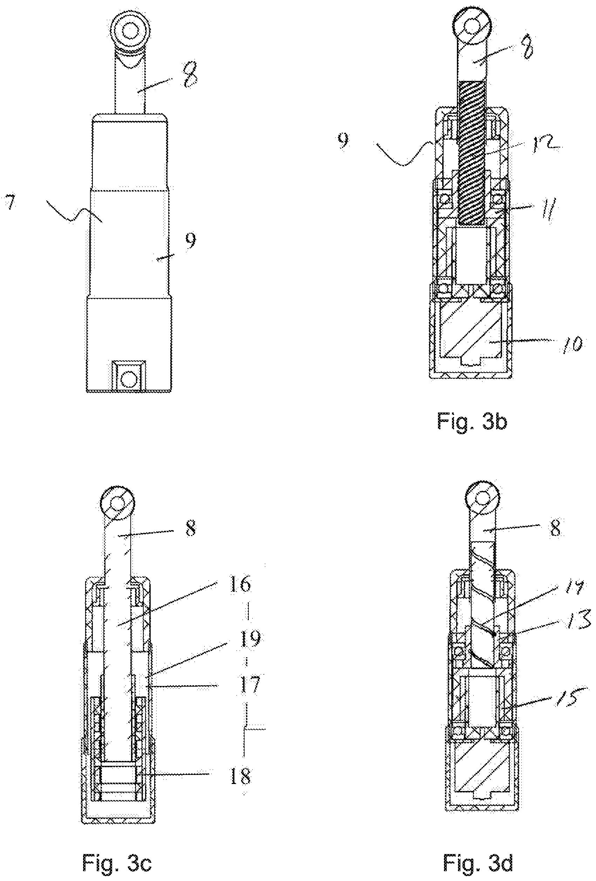

The single sided knee joint can advantageously be operated by a motor as described with reference to figs. 3 a-d. Fig. 3a shows a view of the actuator 7 comprising a housing and a shaft. Fig. 3b shows a sectional view of the actuator 7 comprising an electric motor 10. Fig. 3c shows a sectional view of the actuator 7 comprising a linear motor 17. Fig 3d shows the actuator 7 comprising an electric motor that rotates a ball screw nut and moves a ball screw shaft 8 linearly, or the shaft 8 is moved linearly and generates energy. The actuator 7 also comprises a power source, for instance a battery, that is charged by conventional techniques, for example wired or wireless charging. In some embodiments, the power source can be charged while the user is actively using the prosthesis.

One implementation of a charging system uses excess kinetic energy generated when the user moves to charge the power source. After a movement has been initiated and sufficient help, in the form of electrical energy, has been provided from the motor, the motor will change mode and start to work as a generator. The microprocessor system controls the mode of the motor in the actuator and can change it from working as a motor to working as a generator, as well as change it from working as a generator to work as a motor. When the motor starts working as a generator, voltage is induced in the motor, which is then transferred to a power source. The power source which be an internal battery or an external unit that contains a power source like a battery When kinetic energy is converted to electrical energy and is transferred from the motor to the power source, motion of the joint will slow down. The more energy is transferred, the faster movement of the joint will slow down.

A first embodiment of the actuator 7 is shown in fig. 3b and is an actuator comprising a shaft 8 that moves in the axial direction of the shaft 8 and a housing 9. The housing 9 protects the electric motor 10. The electric motor 10 turns a nut 11 and the shaft 8 moves in the axial direction, and then changes the angular velocity of the artificial knee joint. A thread 12 on the shaft 8 has a high pitch. If the shaft 8 drives back and forth, the shaft 8 will rotate the nut 11. If the nut 11 is rotating without help of the motor, for instance when a user moves the leg without help from the power source, it will spin the electric motor that will produce electric energy when the shaft 8 is moved. In embodiments incorporating a chargeable power source, this electric energy can be used to charge the power source.

Fig. 3c shows a sectional view of a second embodiment of the actuator 7 comprising a linear motor 17. The actuator 7 can also for example comprise an axle 16 that is included in the linear motor 17, which is made up of solenoids 18 and magnets 19 that move the axle in a linear direction, and if the axle is moved linearly it induces electric energy in the linear motor 17.

Fig. 3d shows a third embodiment of the actuator 7 comprising an electric motor that rotates a ball screw nut 13 and moves a ball screw shaft 8 linearly, or the ball screw is moved linearly and the motor generates energy. The nut can also for example be a ball nut to reduce friction between a thread 14 and a nut. The coupling between the nut and the motor can also for example be a free needle hub bearing coupling 15. However, the invention is not limited to a certain type of drive motor, for example a belt motor drive or a hollow shaft motor may also be used.

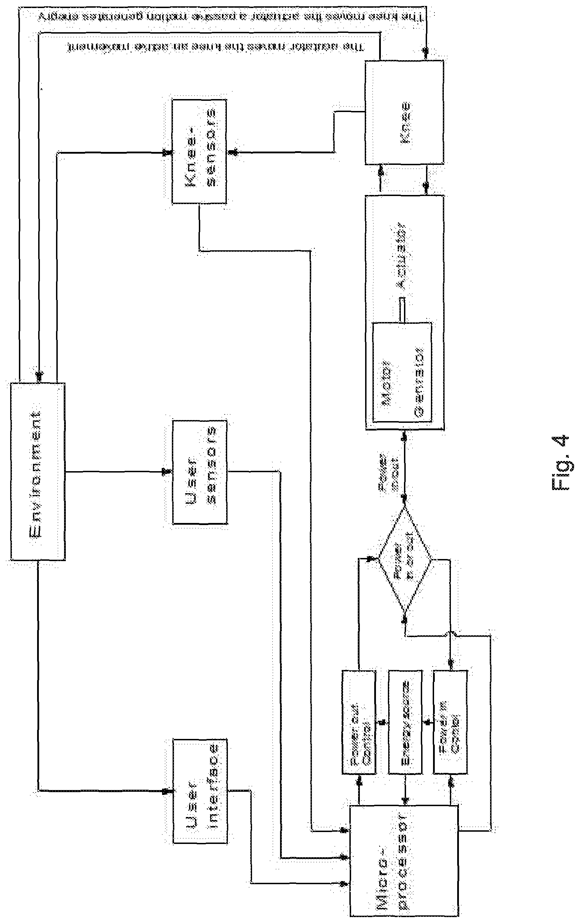

Fig. 4 shows a flow chart describing the operation of the knee joint of the present invention using an electric control system. The microprocessor measures all sensors at least at 1 kHz and decides if the knee shall flex or extend as well as how fast the motions should be. The microprocessor then controls the motor in the actuator to work as a motor consuming energy or generating energy depending on the angular velocity of the prosthetic knee joint. When the prosthetic knee joint starts its flexion or extension motion the angular velocity is low. The microprocessor then actively controls the motor in the actuator to increase the angular velocity and the motor consumes energy. Almost immediately the angular velocity becomes sufficient to perform the required motion, and then the microprocessor controls the motor in the actuator to work as a generator that generates energy instead. When the actuator controls the motor to work as a generator the angular velocity of the prosthetic knee joint will decrease until the extension or flexion motion ends.

The electronic control system uses friction and/or an electric actuator 7 which controls the knee joint flexion and extension movement. The actuator 7 actively controls the angular velocity of the knee joint in all positions. Everything from increasing the knee joint movement to decreasing the movement in the knee joint is controlled. When the knee is controlled with the electric actuator 7 the actuator actively alternates between increasing and decreasing the knee joint angular velocity to mimic the natural movements in an artificial leg. The knee joint prosthesis moves in an anatomically correct manner at different angles, speeds and situations. Furthermore, it is possible to incorporate safety features for the knee joint through the actuator 7. Because the knee sensors values are read at least at 1 kHz, the prosthetic joint can be controlled by the user in real time with virtually no delay. In some embodiments the means for controlling the joint can be in the form of an external control device, such as a remote control or a smartphone. This further facilitates the usage of a prosthetic knee compared to an implementation that only is controllable from where the motor is placed. For example, if a user would need to walk a set of stairs with many steps, a pause might be required. With the help of an external control device it would be possible, with only the click of a button, to change the mode of a prosthetic joint to a locked position when the user wishes to rest, such as in the middle of a set of stairs.

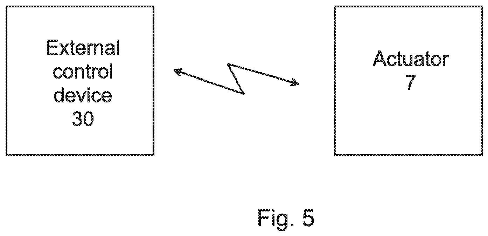

Fig. 5 shows an external control device that can be used to control the actuator which in turn controls the motor. The control device is in a preferred embodiment wirelessly connected to the actuator 7 and can be operated by the user, for instance through hard buttons or through soft buttons embedded in a user interface. The external control device can also be a smartphone, preferably one having a specially designed application for controlling a knee joint prosthesis according to the present invention.

The knee joint prosthesis has at least two sensors detecting what the user wants to do, and what the knee joint is doing. The microprocessor measures and calculates the values in real time and controls the actuator regarding normal use like walking, running, climbing over obstacles, walking backwards, cycling, sitting down etc.

To perform extension and flexion of the knee joint in real time, for example in order to walk over an obstacle, the present invention is controlled by a microprocessor that reads the sensors at least at 1 kHz. The control of the knee joint is based on the collected information. One of the knee sensors measures if the user is loading the prosthetic lower limb. If the limb is loaded, a fast flexion of the knee joint is not desirable because the user may fall. Other sensors may be used for detecting for instance motion, velocity, angular velocity, angular momentum, kinetic energy, amount of energy stored in the power source,

Depending of the load and how fast it changes, the sensor or sensors determine how fast the user moves and the stance phase and swing phase in the gait cycle are controlled accordingly. This enables walking at different speeds forward, backward, indoor, outdoor, off-road, walking over obstacles, stumbling, running, jumping etc.

One advantage with the control means of the present knee joint is that the user sensors are used to determine what the user wants to do. For instance, the sensors can pick up on signals, such as the walk of a person slowing down, that indicate that the user will want to stop soon. This information can be used to, for example, in advance prepare the leg to be in a fully extended mode. This can be done automatically as well as manually by use of for instance an external controller. Another thing that can be determined by the user sensors is if the user will sit or bicycle, in which case the knee shall be very easy to move.

The present invention is designed to operate a prosthetic leg in a variety of modes. Certain of these modes of operation may be directed to achieving a particular gait or to operate the prosthetic leg in a specific manner. Some modes can for example be designed for standing, walking in different speeds, walking forward, backward, walking indoor, outdoor, off-road, walking over obstacles, stumbling, running, jumping etc.

It is possible to produce a prosthetic leg having an electronic control system that operates in only a single mode but preferably, such as in the present invention, the electronic control system is adaptive and can operate the prosthetic leg in a mode selected by the amputee using for instance a remote controller or button located close to the motor, or in a mode automatically selected based on data acquired from sensor measurements of the amputee's activity level and/or of external environmental conditions.

The user interface is a way to align the lower limb prosthetic. It can be changed by a computer, a tablet computer, or an APP on a smart phone. The set up parameters for all different modes makes the lower limb prosthetic work for an individual user. It can be everything from the angular velocity that the user likes during normal gait to which maximal flexion angle the user likes when walking in a slow pace.

While the present description has been described with reference to certain embodiments, it will be understood by those skilled in the art that it can be implemented in similar ways without departing from the scope of the invention.

## Claims

1. A knee joint for use in a lower limb prosthesis comprising:
- an upper portion (50) comprising an attachment part (4) with a first connection means (300) for connecting a socket (22), and
- a support (100) comprising a second connection means (400) in a lower part thereof for connecting a prosthetic limb,
- a joint (2, 3), pivotably interconnecting the upper portion (50) and the support (100), and
- an actuator (7) connected between the attachment part (4) and the support (100) and adapted to pivot the upper portion (50) relatively to the support (100);
- a control unit adapted to control the actuator (7);
**characterized in that**
the joint (2, 3) is provided above the first connection means (300) when the lower limb prosthesis is in normal use.

2. The knee joint according to claim 1, wherein the support (100) comprises a single attachment arm (6) on either the medial or the lateral side of the knee joint prosthesis.

3. The knee joint according to claim 1 or 2, wherein the support (100) is in the form of a vertical attachment arm (6) and a lower horizontal part.

4. The knee joint according to claim 3, wherein the distal end of the vertical attachment arm (6) is located distal to the actuator (7).

5. The knee joint according to any one of claims 1-4, wherein the joint comprises an inner ring (2) and an outer ring (3).

6. The knee joint according to any one of claims 1-5, wherein the joint is one of the following: a slide bearing, a cross roller bearing, a cross needle bearing, and a ball bearing with a deep groove.

7. The knee joint according to any one of claims 1-6, wherein the actuator (7) comprises a motor (10; 17) directly operatively connected to an axially displaceable shaft (8) capable of changing angular velocity of the knee joint.

8. The knee joint according to claim 7, wherein the motor is configured to act both as a motor consuming energy and a generator generating energy.

9. The knee joint according to any one of claims 1-8, comprising at least one sensor connected to the actuator for detecting a parameter of the knee joint,

10. The knee joint according to claim 9, wherein the at least one sensor is adapted to measure at least one of the following: the angular velocity of the knee joint, whether the user is loading the prosthetic lower limb, motion, velocity, momentum, kinetic energy, and amount of energy stored in a power source.

11. The knee joint according to claim 7, wherein the motor is one of the following: an electric motor (10), linear motor (17), a belt motor drive, and a hollow shaft motor.

12. The knee joint according to any one of claims 1-11, wherein the socket is covered with a split cosmetics shell.

13. The knee joint according to any one of claims 1-12, wherein the control unit is adapted to be controlled by means of an external control device (30), preferably a smartphone.

14. The knee joint according to any one of claims 1-13, wherein the knee joint is adapted to operate a prosthetic leg in a variety of modes, preferably directed to achieving a gait, standing, walking in different speeds, walking forward, backward, walking indoor, outdoor, off-road, walking over obstacles, stumbling, running, and/or jumping.

15. A system for providing an amputee with a knee joint for use in a lower limb prosthesis and means for controlling said knee joint, the system comprising:
- a knee joint according to any one of claims 1-14; and
- an external control device (30) wirelessly coupled to the actuator for controlling the movements of the knee joint.

## Patentansprüche

1. Kniegelenk zur Anwendung in einer Prothese für eine untere Extremität, umfassend:
- einen oberen Teil (50), der einen Befestigungsteil (4) mit einem ersten Verbindungsmittel (300) zum Verbinden eines Schafts (22) umfasst, und
- eine Stütze (100), die ein zweites Verbindungsmittel (400) in einem unteren Teil davon zum damit Verbinden einer Prothese umfasst,
- ein Gelenk (2, 3), das den oberen Teil (50) und die Stütze (100) schwenkbar miteinander verbindet, und
- eine Betätigungsvorrichtung (7), die zwischen dem Befestigungsteil (4) und der Stütze (100) verbunden ist und dazu eingerichtet ist, den oberen Teil (50) bezüglich der Stütze (100) zu schwenken;
- eine Steuereinheit, die zur Steuerung der Betätigungsvorrichtung (7) eingerichtet ist;
**dadurch gekennzeichnet, dass**
das Gelenk (2, 3) über dem ersten Verbindungsmittel (300) angebracht ist, wenn die Prothese für eine untere Extremität in normalem Gebrauch ist.

2. Kniegelenk nach Anspruch 1, wobei die Stütze (100) einen einzigen Befestigungsarm (6) entweder auf der medialen oder der lateralen Seite der Kniegelenkprothese umfasst.

3. Kniegelenk nach Anspruch 1 oder 2, wobei die Stütze (100) in Form eines vertikalen Befestigungsarms (6) und eines unteren horizontalen Teils vorliegt.

4. Kniegelenk nach Anspruch 3, wobei das distale Ende des vertikalen Befestigungsarms (6) distal zu der Betätigungsvorrichtung (7) angeordnet ist.

5. Kniegelenk nach einem der Ansprüche 1-4, wobei das Gelenk einen inneren Ring (2) und einen äußeren Ring (3) umfasst.

6. Kniegelenk nach einem der Ansprüche 1-5, wobei das Gelenk eines der folgenden ist: ein Gleitlager, ein Kreuzrollenlager, ein Kreuznadellager und ein Kugellager mit tiefer Rille.

7. Kniegelenk nach einem der Ansprüche 1-6, wobei die Betätigungsvorrichtung (7) einen Motor (10; 17) umfasst, der direkt wirksam mit einer axial verlagerbaren Welle (8) verbunden ist, die die Winkelgeschwindigkeit des Kniegelenks verändern kann.

8. Kniegelenk nach Anspruch 7, wobei der Motor dazu eingerichtet ist, sowohl als Energie verbrauchender Motor als auch als Energie erzeugender Generator zu wirken.

9. Kniegelenk nach einem der Ansprüche 1-8, mindestens einen mit der Betätigungsvorrichtung verbundenen Sensor zum Erfassen eines Parameters des Kniegelenks umfassend.

10. Kniegelenk nach Anspruch 9, wobei der mindestens eine Sensor dazu eingerichtet ist, mindestens eines der folgenden zu messen: die Winkelgeschwindigkeit des Kniegelenks, ob der Benutzer die Prothese für eine untere Extremität belastet, Bewegung, Geschwindigkeit, Schwungkraft, kinetische Energie, und Betrag der in einer Energiequelle gespeicherten Energie.

11. Kniegelenk nach Anspruch 7, wobei der Motor eines der folgenden ist: ein Elektromotor (10), Linearmotor (17), Riemen-Motorantrieb, und ein Hohlwellenmotor.

12. Kniegelenk nach einem der Ansprüche 1-11, wobei der Schaft mit einer geteilten Kosmetikhülle bedeckt ist.

13. Kniegelenk nach einem der Ansprüche 1-12, wobei die Steuereinheit dazu eingerichtet ist, mittels einer externen Steuervorrichtung (30), bevorzugt einem Smartphone, gesteuert zu werden.

14. Kniegelenk nach einem der Ansprüche 1-13, wobei das Kniegelenk dazu eingerichtet ist, eine Beinprothese in einer Vielzahl von Modi zu bedienen, bevorzugt darauf gerichtet, ein Gangbild zu erzielen, Stehen, Gehen in verschiedenen Geschwindigkeiten, Vorwärtsgehen, Rückwärtsgehen, Gehen in Gebäuden, im Freien, im Gelände, Gehen über Hindernisse, Stolpern, Laufen und/oder Springen.

15. System zur Versorgung eines Amputierten mit einem Kniegelenk zur Anwendung in einer Prothese für eine untere Extremität und Mittel zur Steuerung des Kniegelenks, wobei das System umfasst:
- ein Kniegelenk nach einem der Ansprüche 1-14; und
- eine externe Steuervorrichtung (30), die drahtlos mit der Betätigungsvorrichtung gekoppelt ist, zur Steuerung der Bewegungen des Kniegelenks.

## Revendications

1. Articulation de genou destiné à être utilisée dans une prothèse de membre inférieur comprenant:
- une partie supérieure (50) comprenant une partie de fixation (4) avec un premier moyen de connexion (300) pour connecter une douille (22), et
- un support (100) comprenant un second moyen de connexion (400) dans une partie inférieure de celui-ci pour connecter un membre prothétique,
- une articulation (2, 3) interconnectant de manière pivotante la partie supérieure (50) et le support (100), et
- un actionneur (7) connecté entre la partie de fixation (4) et le support (100) et adapté pour faire pivoter la partie supérieure (50) par rapport au support (100);
- une unité de commande adaptée pour commander l'actionneur (7);
**caractérisée en ce que**
l'articulation (2, 3) est prévue au-dessus du premier moyen de connexion (300) lorsque la prothèse de membre inférieur est en utilisation normale.

2. Articulation de genou selon la revendication 1, dans laquelle le support (100) comprend un seul bras de fixation (6) sur le côté soit médial soit latéral de la prothèse d'articulation du genou.

3. Articulation de genou selon la revendication 1 ou 2, dans laquelle le support (100) se présente sous la forme d'un bras de fixation verticale (6) et d'une partie horizontale inférieure.

4. Articulation de genou selon la revendication 3, dans laquelle la partie distale du bras de fixation verticale (6) est située en position distale par rapport à l'actionneur (7).

5. Articulation de genou selon l'une quelconque des revendications 1 à 4, dans laquelle l'articulation comprend une bague intérieure (2) et une bague extérieure (3).

6. Articulation de genou selon l'une quelconque des revendications 1 à 5, dans laquelle l'articulation est l'une des suivantes: un palier coulissant, un roulement à rouleaux croisés, un palier à aiguilles croisées et un roulement à billes avec une rainure profonde.

7. Articulation de genou selon l'une quelconque des revendications 1 à 6, dans laquelle l'actionneur (7) comprend un moteur (10; 17) en communication active directe à un arbre (8) axialement déplaçable capable de changer la vitesse angulaire de l'articulation de genou.

8. Articulation de genou selon la revendication 7, dans laquelle le moteur est configuré pour agir à la fois comme un moteur consommant de l'énergie et un générateur générant de l'énergie.

9. Articulation de genou selon l'une quelconque des revendications 1 à 8, comprenant au moins un capteur connecté à l'actionneur pour détecter un paramètre de l'articulation de genou.

10. Articulation de genou selon la revendication 9, dans laquelle le au moins un capteur est adapté pour mesurer au moins l'un des éléments suivants: la vitesse angulaire de l'articulation de genou, si l'utilisateur charge le membre inférieur de la prothèse, le mouvement, la vitesse, l'élan, l'énergie cinétique et la quantité d'énergie stockée dans une source d'alimentation.

11. Articulation de genou selon la revendication 7, dans laquelle le moteur est l'un des suivants: un moteur électrique (10), un moteur linéaire (17), un moteur à courroie, et un moteur à arbre creux.

12. Articulation de genou selon l'une quelconque des revendications 1 à 11, dans laquelle la douille est recouverte d'une coque cosmétique divisée.

13. Articulation de genou selon l'une quelconque des revendications 1 à 12, dans laquelle l'unité de commande est adaptée pour être commandée au moyen d'une unité de commande externe (30), de préférence un smartphone.

14. Articulation de genou selon l'une quelconque des revendications 1 à 13, dans laquelle l'articulation de genou est adaptée pour faire fonctionner une jambe prothétique dans différents modes, de préférence pour obtenir une démarche, une stature, une marche à différentes vitesses, une marche vers l'avant, vers l'arrière, une marche à l'intérieur, à l'extérieur, hors-route, une marche sur des obstacles, le trébuchement, la course et/ou le saut.

15. Système pour fournir à un amputé une articulation de genou pour une utilisation dans une prothèse de membre inférieur et des moyens pour commander ladite articulation de genou, le système comprenant:
- une articulation selon l'une quelconque des revendications 1 à 14; et
- un dispositif de commande externe (30) couplé sans fil à l'actionneur pour contrôler les mouvements de l'articulation de genou.
